# EUROPÄISCHE PATENTSCHRIFT

(11) **EP 1 125 633 B1**
(45) Veröffentlichungstag und Bekanntmachung des Hinweises auf die Patenterteilung: **03.01.2007**
(21) Anmeldenummer: 01102003.9
(22) Anmeldetag: 30.01.2001
(51) Int. Cl.: B01J 25/00

(54) **Verfahren zur Herstellung von Carbonsäuren durch Dehydrierung von Alkoholen**
Process for preparing carboxylic acids by dehydrogenating alcohols
Procédé de préparation d'acides carboxyliques par la déshydrogénation d'alcools

(30) Priorität: 18.02.2000 EP 00103546
(43) Veröffentlichungstag der Anmeldung: 22.08.2001
(60) Teilanmeldung: 03019556.4
(73) Patentinhaber: Degussa AG, 40474 Düsseldorf (DE)
(72) Erfinder: Berweiler, Monika, 63477 Maintal (DE); Freund, Andreas, Dr., 10606 White Plains, NY (US); Girke, Walter, 63457 Hanau-Grossauheim (DE); Höpp, Matthias, Dr., 36695 Mobile, Alabama (US); Ostgard, Daniel, Dr., 63801 Kleinostheim (DE); Sauer, Jörg, Dr., 36695 Mobile, Alabama (US); Vanheertum, Rudolf Dr., 63796 Kahl (DE)

(56) Entgegenhaltungen:
- EP-A- 1 086 745
- EP-A1- 0 734 765
- EP-A1- 1 067 114
- EP-A2- 0 175 581
- JP-A- 11 279 090
- US-A- 5 292 936
- US-A- 6 028 119
- DATABASE WPI Section Ch, Week 197549 Derwent Publications Ltd., London, GB; Class A41, AN 1975-80884W XP002169056 -& JP 50 093919 A (MITSUI TOATSU CHEM INC) , 26. Juli 1975 (1975-07-26)

## Beschreibung

Die Erfindung betrifft ein Verfahren zur Herstellung von Carbonsäuren aus Alkoholen.

Es ist bekannt, Diethanolamin zu Iminodiessigsäure zu dehydrieren. (US 5,689,000; WO 96/01146; WO 92/06949; JP-OS 091 55 195; US 5,292,936; US 5,367,112; CA 212 10 20)

Gegenstand der Erfindung ist ein Verfahren zur Herstellung von Carbonsäuren aus Alkoholen, wobei man Alkohole, die wenigstens etwas in Wasser löslich sind, in Gegenwart eines Raney-Kupfer Katalysators katalytisch dehydriert, wobei der Alkohol und das resultierende Carboxylat in stark basischer Lösung stabil sind, welches dadurch gekennzeichnet ist, dass der Raney-Kupfer Katalysator mit mindestens einem Metall aus der Gruppe Eisen und/oder Edelmetalle in einer Menge von 10 ppm bis 5 Gew% dotiert ist und eine mittlere Teilchengröße von 35 ±30 µm aufweist.

Die Dotierung kann sowohl durch Zulegierung des Dotierungselements zur Raneylegierung, bestehend aus Kupfer und Aluminium, als auch durch Imprägnierung des fertig präparierten Raney-Kupfers mit dem Dotierungselement bewehrstelligt werden.

Das erfindungsgemäß eingesetzte Raney-Kupfer kann die Dotierungselemente in einer Menge von 10 ppm bis 5 Gew.-% enthalten. Die Edelmetalldotierung kann 10 bis 50.000 ppm, vorzugsweise 500 bis 50.000 ppm, betragen. Die Dotierungsmetalle können aus der Gruppe Eisen sowie Palladium, Platin, Gold, Rhenium, Silber, Iridium, Ruthenium und/oder Rhodium ausgewählt werden.

Das erfindungsgemäß eingesetzte Raney-Kupfer kann Meso- und MakroPoren jedoch keine Mikroporen aufweisen.

Die ursprünglich gebildete Legierung kann mehr als 50 % Kupfer enthalten, sodaß der endgültige Katalysator mehr restliches Aluminium enthält als normalerweise unter denselben Aktivierungsbedingungen gefunden wird.

Die ursprünglich gebildete Legierung kann einer Hitzebehandlung in Luftatmosphäre bei Temperaturen oberhalb von 500 °C vor der Aktivierung unterzogen werden.

Die ursprünglich gebildete Legierung kann mehr als 50 % Kupfer enthalten und in Luftatmosphäre einer Hitzebehandlung bei Temperaturen oberhalb 500 °C vor der Aktivierung unterzogen werden.

Die mittlere Teichengröße des erfindungsgemäße eingesetzte Raney-kupfers ist von Bedeutung bei der Anwendung bei Oxidationsreaktionen beziehungsweise Dehydrierungsreaktionen von A1-koholen.

Das bekannte Raney-Kupfer bildet bei der mehrfachen Verwendung Granulate (Agglomerate), wodurch das Raney-Kupfer desaktiviert wird.

Das erfindungsgemäß eingesetzte, mit Eisen und/oder Edelmetall dotierte Raney-Kupfer wird nicht durch eine unerwünschte Granulation desaktiviert. Das erfindungsgemäße eingesetzte Raney-Kupfer läßt sich vorteilhafterweise gut filtrieren.

Das erfindungsgemäß eingesetzte Raney-Kupfer zeigt eine höhere Aktivität als das Cr/Raney-Kupfer gemäß EP 0 620 209 A1 oder US 5,292,936 bei der Dehydrierung von Ethylenglykol.

Das erfindungsgemäße Raney-Kupfer enthält weiterhin vorteilhafterweise keine toxischen Metalle, wie zum Beipiel Chrom.

Das Raney-Kupfer, kann hergestellt werden, in dem man eine Kupfer-Aluminium-Legierung mittels wäßriger Natronlaugenlösung aktiviert, den Katalysator wäscht, in Wasser suspendiert, zu dieser Suspension eine Eisensalz- oder Edelmetallsalz-Lösung hinzugibt, den pH-Wert der Lösung auf einen Wert von 4 bis 11 einstellt, den Katalysator von der Lösung abtrennt und wäscht. Weiterhin kann man das Raney-Kupfer herstellen, indem man das Dotierungsmetall zusammen mit Kupfer und Aluminium legiert, anschließend mittels wäßriger Natronlaugenlösung aktiviert und den Katalysator wäscht.

Das erfindungsgemäße Verfahren zur Dehydrierung von Alkoholen zu den entsprechenden Carbonsäuren kann zur Dehydrierung von Glykolen und/oder Aminoalkoholen verwendet werden. Dabei kann der Katalysator in Form einer Suspension eingesetzt werden.

Die Alkohole, die erfindungsgemäß dehydriert werden können, können ein- oder mehrwertige Alkohole sein. Sie können inklusive von Polyetherglykolen aliphatische, cyclische oder aromatische Verbindungen sein, die mit einer starken Base zu dem Carboxylat reagieren.

Hierbei ist es notwendig, daß der Alkohol und das resultierende Carboxylat in stark basischer Lösung stabil sind, und der Alkohol wenigstens etwas in Wasser löslich ist.

Geeignete primäre, einwertige Alkohole können umfassen:

Aliphatische Alkohole, die verzweigte, gradkettige, cyclische oder aromatische Alkohole, wie zum Beispiel Benzylalkohol, sein können, sein, wobei diese Alkohole mit verschiedenen, basenstabilen Gruppen substituiert sein können.

Geeignete aliphatische Alkohole können Ethanol, Propanol, Butanol, Pentanol oder ähnlich sein.

Erfindungsgemäß können Glykole zu Carbonsäuren oxidiert beziehungsweise dehydriert werden. Glykole können zum Beispiel sein:
Ethylenglykol
Propopylenglykol
1,3-Propandiol
Butylenglykol
Butandiol -1,4

So kann man zum Beispiel Ethylenglykol zu Glykolsäure (Monocarbonsäure) dehydrieren und durch anschliessende Umsetzung mit KOH die Dicarbonsäure Oxalsäure herstellen.

Aminoalkohole können ebenfalls mit dem erfindungsgemäß eingesetzten, dotierten Raney-Kupfer zu den entsprechenden Aminocarbonsäuren dehydriert werden. Die Aminoalkohole können 1 bis 50 C-Atome aufweisen.

So kann man beispielsweise
N-Methylethanolamin zu Sarkosin; THEEDA (Teträhydroxyethylenthylendiamin)zum Tetranatriumsals von EDTA (Ethylendiamtetraacetat);
Monoethanolamin zu Glycin;
Diethanolamin zu Iminodiessigsäure;
3-Amino-1-propanol zu Beta-Alanin;
2-Amino-1-butanol zu 2-Aminobuttersäure dehydrieren.

In einer Ausführungsform der Erfindung können mit dem erfindungsgemäßen Verfahren Aminoalkohole der Formel 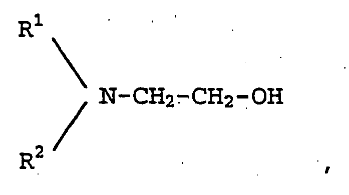 in der R¹ und R² jeweils Wasserstoff; Hydroxyethyl; - CH₂CO₂H; eine Alkylgruppe mit 1 bis 18 C-Atomen; eine Aminoalkylgruppe mit 1 bis 3 C-Atomen; eine Hydroxyalkylaminoalkyl-Gruppe mit 2 bis 3 C-Atomen sowie Phosphonomethyl bedeuten, dehydriert werden.

Die Aminoalkohole, die erfindungsgemäß eingesetzt werden können, sind bekannt. Wenn R¹ und R² gleich Wasserstoff sind, dann ist der Aminoalkohol Diethanolamin.

Wenn R¹ und R² Hydroxyethyl sind, dann ist der Aminoalkohol Triethanolamin. Die resultierenden Aminocarbonsäuresalze dieser Ausgangsaminoalkohole sollten die Salze von Glycin, Iminodiessigsäure, beziehungsweise Nitrilotriessigsäure sein. Weitere Aminoalkohole umfassen N-methylethanolamin, N,N-dimethylethanolamin, N-ethylethanolamin, N-isopropylethanolamin, N- butylethanolamin, N-nonylethanolamine, N-(2-aminoethyl)ethanolamin, N-(3-aminopropyl)ethanolamin, N,N-diethylethanolamin, N,N-dibutylethanolamin, N-methyldiethanolamin, N-ethyldiethanolamin, N-isopropyldiethanolamin, N-butyldiethanolamin, N-ethyl,N-(2-aminoethyl)-ethanolamin, N-methyl,N-(3-aminopropyl)ethanolamin, tetra(2-hydroxyethyl)ethylenediamin, und ähnliche.

Weitere Beispiele für Aminocarbonsäuresalze sind die Salze von N-methylglycin, N,N-dimethylglycin,N-ethylglycin, N-isopropylglycin, N-butylglycin, N-nonylglycin, N-(2-aminoethyl)glycin, N-3-aminopropyl)glycin, N,N-diethylglycin, N,N-dibutylglycin, N-methyliminodiessigsäure, N-ethyliminodiessigsäure, N-isopropyliminodiessigsäure, N-butyliminodiessigsäure, N-ethyl, N-(2-aminoethyl)glycin, N-methyl-N-(3-aminopropyl)glycin, Ethylenediaminetetraessigsäure, und so weiter.

R¹ oder R² können ebenfalls eine Phosphonomethyl-Gruppe sein, wobei die Ausgangsaminoverbindung N-phosphonomethylethanolamin und die resulierend Aminosäure N-phosphonomethylglycin sein können. Wenn von R¹ oder R² ein R = Phosphonomethyl und das andere R = -CH₂CH₂OH ist, wäre die resultierende Aminosäure N-phosphonomethyliminodiessigsäure, die zu N-phosphonomethylglycin auf bekanntem Wege umgewandelt werden kann. Wenn von R¹ oder R² ein R = Phosphonomethyl ist und das andere R = eine Alkylgruppe ist, wäre die resultierende Säure N-alkyl-N-phosphonomethylglycin, das zu N-phosphonomethylglycin entsprechend dem U.S. Patent 5,068,404 weiter umgewandelt werden kann.

Das erfindungsgemäße Verfahren kann bei einer Temperatur von 50 bis 250 °C, bevorzugt 80 bis 200 °C, und bei einem Druck von 0,1 bis 200 bar, bevorzugt Normaldruck bis 50 bar, durchgeführt werden.

Der Druck ist notwendig, weil die Alkohole einen hohen Dampfdruck aufweisen. Bei dem Ablassen des Wasserstoffes würde bei einem zu niedrigen Druck auch der Alkohol abgelassen werden.

### Beispiel 1: (Herstellung des erfindungsgemäß eingesetzten Katalysators)

Eine Legierung, bestehend aus 50%Cu/50%Al wird mit einer wässrigen Natronlaugelösung aktiviert. Der entsprechende Katalysator wird gewaschen bis das Natriumaluminat vollständig entfernt ist. Anschließend wird Hexachloroplatin zu der Suspension des gewaschenen Katalysators gegeben. Der pH-Wert wird eingestellt und die Suspension weiter gerührt. Der dotierte Katalysator wird anschliessend gewaschen. Der Platingehalt des Katalysators ist 1%. Die Aktivität dieses Katalysator für Ethyleneglycoldehydrierung ist 299 ml Wasserstoff pro Stunde pro Gram Katalysator (siehe Beispiel 3).

### Beispiel 2: (Herstellung des erfindungsgemäß eingesetzten Katalysators)

Eine Legierung, bestehend aus 50%Cu/50%Al, wird mit einer wässrigen Natronlaugelösung aktiviert. Der entsprechende Katalysator wird gewaschen bis das Natriumaluminat vollständig entfernt ist. Anschließend wird Eisen III Chlorid zu der Suspension des gewaschenen Katalysators gegeben. Der pH-Wert wird eingestellt und die Suspension weiter gerührt. Der dotierte Katalysator wird anschliessend gewaschen. Der Eisengehalt des Katalysators ist 3%.

### Beispiel 3

Die Dehydrierung von Ethylenglycol zu Natriumglycolat und Natriumoxalat mittels dem aktivierten Kupferkatalysator gemäß Beispiel wird bei 108°C und atmosphärischem Druck durchgeführt. Zuerst wird 70 ml Ethylenglycol zu einer heterogenen Suspension von 8 Gramm Katalysator und 70 ml einer wässerigen Natronlaugelösung gegeben. Die Suspension wird bei 400 upm gerührt. Die Reaktionsgeschwindigkeit wird an Hand der entwickelten Menge Wasserstoff zwischen 30 und 90 Minuten seit Beginn der Reaktion gemessen. Die Ergebnisse werden als ml Wasserstoff pro Stunde pro Gramm Katalysator angegeben. Die Aktivität dieses Katalysators für die Dehydrierung von Ethylenglykol ist 299 ml Wasserstoff pro Stunde pro Gramm Katalysator.

### Beispiel 4 (Vergleichsbeispiel)

Eine Legierung, bestehend aus 50%Cu/50%Al, wird mit einer wässrigen Natronlaugelösung aktiviert. Der entsprechende Katalysator wird gewaschen, bis das Natriumaluminat vollständig entfernt ist. Die Aktivität dieses Katalysators ist bei der Ethylenglycoldehydrierung 205 ml Wasserstoff pro Stunde pro Gramm Katalysator.

### Beispiel 5 (Vergleichsbeispiel)

Eine 50&Cu/50%Al Legierung wird mit einer wässerigen Natronlaugelösung aktiviert. Der entsprechende Katalysator wird gewaschen, bis das Natriumaluminat vollständig entfernt ist. Chromnitrat wird zu der Suspension des gewaschenen Katalysators gegeben, der pH-Wert wird reguliert, die Suspension weiter gerührt und der dotierte Katalysator noch einmal gewaschen. Der Gehalt an Chrom in dem Katalysator ist 2000 ppm. Die Aktivität des Katalysators bei der Ethylenglycoldehydrierung ist 253 ml Wasserstoff pro Stunden pro Gram Katalysator.

### Beispiel 6 (Vergleichsbeispiel)

Eine Cu/A1/V Legierung wird mit einer wässerigen Natronlaugenlösung aktiviert. Der entsprechende Katalysator wird gewaschen, bis das Natriumaluminat vollständig entfernt ist. Der Gehalt an V in dem Katalysator beträgt 1%. Die Aktivität des Katalysators bei der Ethylenglycoldehydrierung ist 253 ml Wasserstoff pro Stunde pro Gram Katalysator.

### Beispiel 7

### Herstellung von Iminodiessigsäure mit Platin auf Raney-Kupfer als Katalysator.

Das Beispiel illustriert die Umsetzung von Diethanolamin (DEA) zum Natrium-Salz der Iminodiessigsäure (IDA) mit Pt-dotiertem Raney-Kupfer als Katalysator.

Die Versuche werden in einem 2 1 Autoklav (Fa. Büchi) durchgeführt. Der Autoklav ist mit einem Begasungsrührer ausgestattet, der standardmäßig bei 500 min-1 betrieben wird. Der Autoklav ist mit einem Doppelmantel ausgestattet. Die Temperatur im Autoklaven kann über einen Öl-Thermostaten eingestellt werden.

In dem Autoklaven wird folgender Ansatz vorgelegt:

| | |
|---|---|
| 318.8 g | Diethanolamin (3 mol) |
| 508 g | wässrige NaOH-Lösung (50 Gew.-%, 6.3 mol NaOH) |
| 64 g | erfindungsgemäß eingesetzter Katalysator: 1% Pt auf Raney-Kupfer, unter Wasser gelagert |
| 370 g | H₂O, ultraschall-entgast |

Der Autoklav wird mit Stickstoff auf 10 bar aufgedrückt und auf Reaktionstemperatur gebracht (TR=160°C). Nach Anspringen der Reaktion wird der entstehende Wasserstoff abgelassen, wobei die freigesetzte Menge über eine Trocken-Gasuhr bestimmt wird. Die Reaktion wird nach einer Dauer von 5 h abgebrochen und der Autoklav abgekühlt. Die Reaktionsprodukte werden mit entgastem Wasser aus dem Autoklaven gespült, der Katalysator abfiltriert und die Produkte der Dehydrierung durch ionenchromatographische Auftrennung analysiert.

Der eingesetzte Katalysator kann - wie in der Tabelle 1 dargestellt ist - ohne nennenswerten Aktivitätsverlust mehrfach rezykliert werden.

**Tabelle 1 Umsetzung von Diethanolamin an Pt-dotiertem Raney-Kupfer**

| **Anzahl der Ansätze mit Katalysator** | **Ausbeute IDA [mol %]** |
|---|---|
| 1 | 94.3 |
| 2 | 92.5 |
| 3 | 98.6 |
| 4 | 96.8 |
| 5 | 95.0 |
| 6 | 94.7 |
| 7 | 90.9 |
| 8 | 91.8 |
| 9 | 93.4 |
| 10 | 95.8 |
| 11 | 97.7 |
| 12 | 93.5 |
| 13 | 95.7 |
| 14 | 92.6 |
| 15 | 90.0 |
| 16 | n.b. |
| 17 | n.b. |
| 18 | 95.2 |

### Beispiel 6

### Herstellung von Iminodiessigsäure mit Eisen auf Raney-Kupfer als Katalysator.

In einem 2 l Autoklaven wird folgender Ansatz vorgelegt:

| | |
|---|---|
| 318.8 g | Diethano,lamin (3 mol) |
| 508 g | wässrige NaOH-Lösung (50 Gew.-%, 6.3 mol NaOH) |
| 64 g | erfindungsgemäß eingesetzter Katalysator: 3% Fe auf Raney-Kupfer, unter Wasser gelagert |
| 370 g | H₂O, ultraschall-entgast |

Der Versuch wird analog zu Beispiel 5 durchgeführt. Dabei werden die in Tabelle 2 aufgelisteten Ausbeuten erzielt, Eine Desaktivierung des Katalysators ist auch nach mehrfachem Einsatz des Katalysators nicht zu beobachten

**Tabelle 2 Umsetzung von Diethanolamin an Fe-dotiertem Raney-Kupfer**

| **Anzahl der Ansätze mit Katalysator** | **Ausbeute IDA [mol %]** |
|---|---|
| 1 | 95.3 |
| 2 | 99.1 |
| 3 | 99.0 |
| 4 | n.b. |
| 5 | n.b. |
| 6 | 91.9 |
| 7 | n.b. |
| 8 | n.b. |
| 9 | n.b. |
| 10 | 93.7 |
| 11 | n.b. |
| 12 | n.b. |
| 13 | n.b. |
| 14 | 94.0 |

### Beispiel 7

### Vergleichsbeispiel

### Herstellung von Iminodiessigsäure an undotiertem Raney-Kupfer.

Reines Raney-Kupfer (Degussa-Katalysator BFX 3113W) wird unter den Bedingungen von Beispiel 5 eingesetzt. Das Raney-Kupfer zeigt schon nach wenigen Ansätzen eine deutliche Deaktivierung. (Tabelle 3)

**Tabelle 3 Umsetzung von Diethanolamin an Raney-Kupfer**

| **Anzahl der Ansätze mit Katalysator** | **Ausbeute IDA [mol %]** |
|---|---|
| 1 | 91.6 |
| 2 | 82.8 |
| 3 | 68.3 |
| 4 | 51.3 |

### Beispiel 8

### Herstellung von Glycin mit Platin auf Raney-Kupfer als Katalysator.

In dem 2 1 Autoklaven wird folgender Ansatz vorgelegt:

| | |
|---|---|
| 307 g | Monoethanolamin (5 mol) |
| 420 g | wässrige NaOH-Lösung (50 Gew.-%, 5.25 mol NaOH) |
| 64 g | erfindungsgemäß eingesetzter Katalysator: 1% Pt auf Raney-Kupfer, unter Wasser gelagert |
| 400 g | H₂O; ultraschall-entgast |

Der Versuch wird analog zu Beispiel 5 durchgeführt. Dabei werden die in Tabelle 4 aufgelisteten Ausbeuten erzielt. Eine Desaktivierung des Katalysators ist auch nach mehrfachem Einsatz des Katalysators nicht zu beobachten.

**Tabelle 4 Umsetzung von Monoethanolamin an Pt-dotiertem Raney-Kupfer**

| **Anzahl der Ansätze mit Katalysator** | **Ausbeute Glycin [mol %]** |
|---|---|
| 1 | 98.5 |
| 2 | 97.5 |
| 3 | n.b. |
| 4 | n.b. |
| 5 | 98.1 |

### Beispiel 9

### Herstellung von β-Alanin mit Platin auf Raney-Kupfer als Katalysator.

In dem 2 l Autoklaven wird folgender Ansatz vorgelegt:

| | |
|---|---|
| 380 g | 3-Amino-1-propanol (5 mol) |
| 422 g | wässrige NaOH-Lösung (50 Gew.-%, 5.25 mol NaOH) |
| 64 g | erfindungsgemäß eingesetzter Katalysator: 1% Pt auf Raney-Kupfer, unter Wasser gelagert |
| 250 g | H₂O; ultraschall-entgast |

Der Versuch wird analog zu Beispiel 5 durchgeführt. Dabei werden die in Tabelle 5 aufgelisteten Ausbeuten erzielt. Eine Desaktivierung des Katalysators ist auch nach mehrfachem Einsatz des Katalysators nicht zu beobachten.

**Tabelle 5 Umsetzung von 3-Amino-l-propanol an Pt-dotiertem Raney-Kupfer**

| **Anzahl der Ansätze mit Katalysator** | **Ausbeute β-Alanin [mol %]** |
|---|---|
| 1 | 98.2 |
| 2 | 98.5 |
| 3 | n.b. |
| 4 | n.b. |
| 5 | 98.3 |

### Beispiel 10

### Herstellung von 2-Amino-Buttersäure mit Platin auf Raney-Kupfer als Katalysator.

In dem 2 l Autoklaven wird folgender Ansatz vorgelegt:

| | |
|---|---|
| 460 g | 2-Amino-1-butanol (5 mol) |
| 392 g | wässrige NaOH-Lösung (50 Gew.-%, 5.25 mol NaOH) |
| 64 g | erfindüngsgemäß eingesetzter Katalysator: 1% Pt auf Raney-Kupfer, unter Wasser gelagert |
| 140 g | H₂O; ultraschall-entgast |

Der Versuch wird analog zu Beispiel 5 durchgeführt. Dabei werden die in Tabelle 6 aufgelisteten Ausbeuten erzielt. Eine Desaktivierung des Katalysators ist auch nach mehrfachem Einsatz des Katalysators nicht zu beobachten.

**Tabelle 6 Umsetzung von 2-Amino-1-butanol an Pt-dotiertem Raney-Kupfer**

| Anzahl der Ansätze mit Katalysator | Ausbeute 2-Amino-1-Buttersäure [mol %] |
|---|---|
| 1 | 99.2 |
| 2 | 98.1 |
| 3 | n.b. |
| 4 | n.b. |
| 5 | 98.9 |

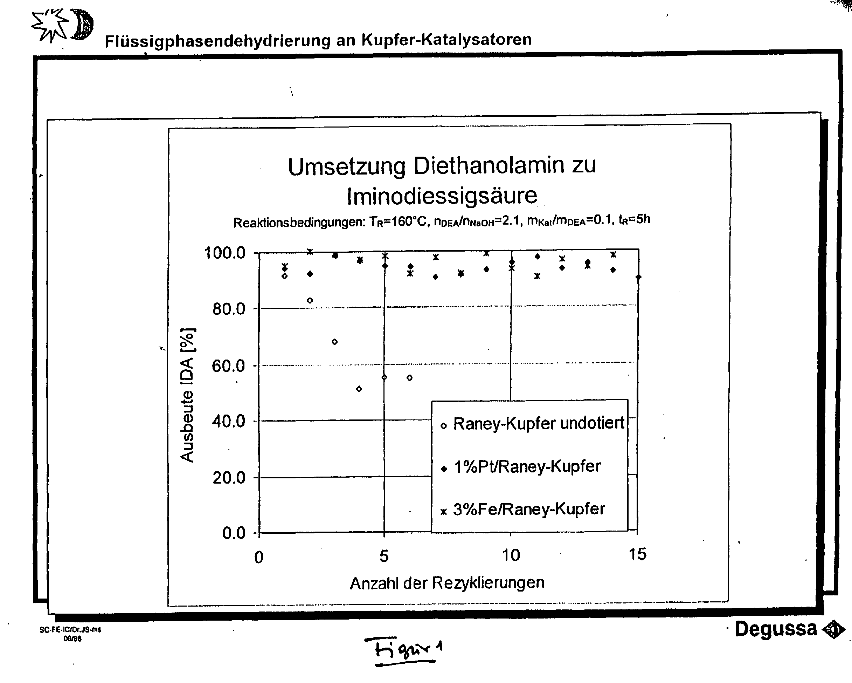

**Figur 1** zeigt den Vorteil des erfindungsgemäß eingesetzten Katalysators am Beispiel der Dehydrierung beziehungsweise der Umsetzung von Diethanolamin zu Iminodiessigsäure

Der erfindungsgemäß eingesetzte Katalysator zeigt eine deutlich höhere Standzeit als der undotierte Raney-Katalysator.

## Patentansprüche

1. Verfahren zur Herstellung von Carbonsäuren aus Alkoholen, wobei man Alkohole, die wenigstens etwas in Wasser löslich sind, in Gegenwart eines Raney-Kupfer Katalysators katalytisch dehydriert, wobei der Alkohol und das resultierende Carboxylat in stark basischer Lösung stabil sind,
**dadurch gekennzeichnet, dass** der Raney-Kupfer Katalysator mit mindestens einem Metall aus der Gruppe Eisen und/oder Edelmetalle in einer Menge von 10 ppm bis 5 Gew% dotiert ist und eine mittlere Teilchengröße von 35 ±30 µm aufweist.

## Claims

1. Process for preparing carboxylic acids from alcohols which comprises alcohols that are at least somewhat soluble in water being catalytically dehydrogenated in the presence of a Raney copper catalyst, the alcohol and the resulting carboxylate being stable in a strongly basic solution,
**characterized in that** the Raney copper catalyst has been doped with at least one metal from the group consisting of iron and/or noble metals in an amount of 10 ppm to 5% by weight and has an average particle size of 35 ± 30 µm.

## Revendications

1. Procédé de préparation d'acides carboxyliques à partir d'alcools, dans lequel des alcools, qui sont au moins un peu solubles dans l'eau, sont soumis à une déshydratation catalytique en présence d'un catalyseur au cuivre de Raney, l'alcool et le carboxylate résultant étant stables dans une solution fortement basique,
**caractérisé en ce que** le catalyseur au cuivre de Raney est dopé avec au moins un métal parmi le groupe constitué du fer et/ou de métaux précieux, en une quantité de 10 ppm à 5 % en poids, et présente une granulométrie moyenne de 35 ± 30 µm.
